# EUROPEAN PATENT SPECIFICATION

(11) **EP 2 298 212 B1**
(45) Date of publication and mention of the grant of the patent: **15.10.2014**
(21) Application number: 10180552.1
(22) Date of filing: 27.07.2004
(51) Int. Cl.: A61B 18/22, A61F 9/008

(54) **Illumination and laser source**
Beleuchtung und Laserquelle
Éclairage et source laser

(30) Priority: 28.07.2003 US 490399 P; 05.03.2004 US 550979 P; 05.06.2004 US 577740 P; 05.06.2004 US 577618 P; 27.07.2004 US 900939
(43) Date of publication of application: 23.03.2011
(62) Divisional of application: 04786120.8
(73) Proprietor: Synergetics, Inc., O'Fallon, MO 63368 (US)
(72) Inventor: Auld, Michael D., Flemington NJ 08822 (US); Easley, James C., O'Fallon, MO 63368 (US); Kane, Jonathan S., Hudson, NH 03051 (US); Scheller, Gregg, Wildwood, MO 63038 (US)
(74) Representative: Hano, Christian

(56) References cited:
- US-A- 6 069 689

## Description

### BACKGROUND OF THE INVENTION

The art of the present invention relates to fiberoptic endoscopic probes for vitreoretinal surgery in general and more particularly to an apparatus and method for delivery of both broad spectrum illumination and coherent laser treatment pulses through a common optical fiber.

Prior art vitreoretinal surgical procedure utilizes discrete and separate optical fibers for the delivery of typically non-coherent light for illumination and coherent laser beam light for surgical treatment of tissues. Although prior art "illuminated laser probes" of various configurations have been developed, they all utilize separate optical fiber or fibers for the non-coherent illumination stream and the coherent laser delivery. The aforesaid fibers are typically arranged side by side inside of a common needle lumen. An embodiment of this prior art technology is found in U.S. Patent # 5,323,766, issued to Uram. This prior art technology requires a larger or more than one incision in order to introduce illumination and laser treatment light into the eye or other structure, thereby generating greater trauma to the surgical site.

Prior art devices typically utilize a laser deliver core optical fiber diameter of typically 200 to 300 microns since said diameter provides the surgical laser burn spot size most commonly desired by the surgeon. The aforesaid prior art devices have been unable to provide sufficient surgically useful illumination (non-coherent white light) power through such a small fiber, primarily due to the prior art's inability to focus said non-coherent surgically useful light onto such a small spot size. Moreover, none of the prior art devices have combined the aforesaid surgically useful illumination and laser treatment light and transmitted through a single fiber, especially of the aforesaid small size.
The present art apparatus and method provides coaxial delivery of both broad spectrum illumination and coherent laser treatment pulses through a common optical fiber. In a preferred embodiment, the apparatus first comprises a non-coherent light source (coherent in an alternative embodiment) capable of coupling sufficient illumination light into an optical fiber with a core diameter suitable for vitreoretinal laser treatment light delivery. That is, to provide a volume of light to the surgical site which is sufficient for illumination of the surgical procedure. In a preferred embodiment said core fiber diameter is typically 200 to 300 microns since said diameter provides the surgical laser bum spot size most commonly desired by the surgeon. The aforesaid optical fiber is typically a multi-mode stepped index fiber in a preferred embodiment. Alternative embodiments may vary the type and size of the optical fiber without departing from the scope of the present art. An object of the present invention is to utilize a light source capable of using 250 micron (or smaller) optical fibers while still providing similar surgically useful lumen output to current 750 micron fiber sources (typically 10-12 lumens). The source output aperture of the present invention in a preferred embodiment is at least 0.5 na (numerical aperture). Alternative embodiments may vary this numerical aperture without departing from the scope of the present invention. The color of the light delivered by the present invention appears white despite the light power output or intensity. Also, the output intensity is capable of reduction without significantly affecting the color, aperture, or homogeneity of the light. The output bandwidth of the aforesaid light is substantially limited to the visible spectrum, that is both UV and IR light are minimized. An option for user selectable limitations (separate from the UV and IR limitations) in the output spectrum is provided. Apparatus conformance to relevant safety standards is also provided.

Prior art illumination light sources typically require a minimum aggregate optical fiber core area equivalent to a fiber diameter of approximately 500 microns in order to deliver sufficient illuminating light to be considered useful by the surgeon. A fundamental prior art limitation with utilization of smaller light fibers for illumination is the size of the focus spot in the light source itself. In a preferred embodiment, the art of the present invention utilizes a small geometry arc lamp which is capable of focusing to an extremely small illumination spot size due to its extremely small plasma ball. This focusing attribute allows for efficient coupling of illumination light into an optical fiber of 100 to 300 micron core diameter which is typically utilized for laser treatment light delivery. Utilization of the aforesaid preferred embodiment allows for up to 40 milliwatts of illumination light to be delivered by a fiber previously considered too small to be an efficient illumination light source. The aforesaid present art light source includes an input aperture or connector for the attachment of a laser coupling fiber. The aforesaid aperture attachment is somewhat similar to the method by which a treatment laser is attached with an ophthalmic slit lamp. That is, via a fiber optic pigtail typically equipped with a mechanical output connector such as an exisma. In the preferred embodiment, dichroic optics and/or other optical path design techniques are used to coaxially couple a treatment laser beam into the illumination optical path, and into an endoscopic probe optical fiber. That is, with the aforesaid coupling arrangement (using a single fiber), the present art apparatus and method allows a unique single and smaller optical fiber to be utilized for both illumination and laser treatment purposes. The art of the present invention further provides a new generation of vitreo-retinal endoscopic instrumentation which utilizes the prior art space occupied by larger illumination fibers and is also capable of providing such in a smaller cross-sectional fiber bundle.

The present art accepts laser light from various surgical laser sources, mixes said laser light with illumination light, and launches both down a single fiber. Laser output aperture is minimized and the laser light is not substantially affected by the illumination dimming or other spectral output limiting. An aiming beam is visible within the illumination output pattern. Unique to the present art is a shadow appearance in the output light cone which indicates the location of laser treatment upon activation of a laser light source. Power losses through the system are also minimized. As aforesaid, the laser mixing method does not significantly affect illumination when not in use (i.e. color, aperture, or homogeneity).

Another unique feature of the present art invention is the ability to change the angular light output from an endoscopic probe coupled with the aforesaid coaxial optical fiber by actively controlling the focus characteristics of the light source. That is, prior art light sources have a fixed numerical aperture focus configuration which is typically designed to fill the full acceptance cone of the mating optical illumination fiber. The present art invention further comprises and utilizes surgeon controlled condensing optics to provide a variable focused light output from the endoscopic probe and efficient coupling into different fiber types. This is especially useful for coupling with optical fibers having different numerical aperture requirements.

Ophthalmic surgical illumination devices for use with optical fibers are found in the prior art and have been manufactured by numerous companies for years. One of many such devices is described in U.S. Patent #4,757,426 issued to Scheller, et al. on July 12, 1988, Entitled *"Illumination System for Fiber Optic Lighting Instruments".* One of the most widely used illumination devices is the "Millennium" which is manufactured by Bausch and Lomb®. Other manufacturers are Alcon® with the "Accurus" and Grieshaber® with the "GLS150". Due to the prevalence of the aforesaid within the marketplace, it is desirable for new and high intensity illumination devices, such as the present art device, to provide an intensity reference indication to ophthalmic surgeons which allows them to reliably duplicate or mimic the illumination intensity of one or more of the aforesaid prior art devices. This is especially true since retinal photic injury is a possible complication of the need to use bright light to clearly visualize ocular structures during delicate ophthalmic surgical procedures. The present art invention further represents a novel apparatus and method for providing the ophthalmic surgeon with graphical photoxicity risk information in a clear and easy to understand manner. In a preferred embodiment, it is comprised of an inexpensive card that is removably attached to the control panel of a surgical light source in order to show the relationship between the output intensity of the light source and the likelihood of photic injury.
US 6 069 68 A discloses an optical system for diagnosis of skin diseases which uses a quartz tungsten halogen filament lamp as a white light source. The filament lamp comprises a larger fiber bundle. The center fiber of the bundle is a collection fiber for collecting the broad illumination of the bundle. The white light is mixed with the laser light and is indistinguishable at the output. A preferably helium-cadmium laser is introduced for fluorescence through a non-closing 2mm hole in a mirror and a near infrared diode laser is used for the excitation illumination for Raman spectroscopy.

Accordingly, it is an object of the present invention to provide an illumination and laser source which is capable of transmitting both illumination (non-coherent) and laser (coherent) treatment light through a single optical fiber of sufficiently small diameter that said fiber may be used for laser treatment, especially in eye surgical or ophthalmic applications.

Another object of the present invention is to provide an illumination and laser source which provides both a surgically useful illumination (non-coherent) output and a combined laser (coherent) output.

Another object of the present invention is to provide an illumination and laser source with an illumination intensity control which is usable by the surgeon to control illumination intensity without affecting laser output power or laser beam spot size characteristics or illumination spectral content.

A further object of the present invention is to provide an illumination and laser source which connects with conventional laser light sources.

A further object of the present invention is to provide an illumination and laser source which provides a shadow or aiming hole within the illumination light cone projection where the laser treatment is placed.

A yet further object of the present invention is to provide an illumination and laser source which minimizes trauma to the patient and surgical site.
The foregoing objects are achieved by an illumination and laser source comprising the features of claim 1. Preferred embodiments of the illumination and laser source of the present invention are claimed in claims 2 to 13.

The illumination and laser source of the present there is provides non-coherent illumination light and coherent laser treatment light through a single optical fiber of the size typically used for laser treatment only. The apparatus is especially suited for use during ophthalmic surgery.

The present art, in a preferred embodiment, utilizes a 75 watt xenon arc lamp for its high luminance illumination (light density), greater than 6000 °K color temperature, and greater than 95 color rendering index. The xenon arc lamp further provides an extremely small point light source which allows for a smaller output illumination beam diameter. Unique to the present lamp source is a mount which allows for replacement of the lamp and yet retains the location of the plasma ball of said source precisely at a predetermined location within the optical center of the apparatus.

A classic spherical reflector and two lens light collection layout is utilized rather than other lower part count layouts, such as using an elliptical reflector or a combination of a parabolic reflector and lens. Light that is incident on the reflector is reflected back to the lamp. A first achromatic lens collimates light from the source and the upside down or inverted image. A second achromatic lens is located coaxial to the first lens and focuses the light at its focal point. The optical fiber is located at the focal point of the second lens. The aforesaid reflectors are preferably spherical rather than parabolic in order to reflect illumination light in the same form as sourced from the arc lamp.

An additional separate illumination path is possible with the present art. No other conventional illumination light source incorporates multiple light paths from a single lamp. The independent nature of the two paths allow different filtering and intensity control settings to the two outputs.

Output dimming of the present art illumination is accomplished by steering the first (collimating) or penultimate lens in a fashion that does not change the lens numerical aperture or introduce shadow artifacts into the beam. A control knob allows the user to select the desired illumination level by rotating the knob.

The output optical fiber connector is uniquely configured to provide the precise positioning required while reducing cost. A precise connector end is combined with an integral retention thread to reduce parts cost and assembly time. An optional groove or recess is placed on a second version of the connector to provide for sensing the difference between illumination only and laser compatible output fibers. Placement of a smooth diameter connector into the output activates a switch which will allow the laser power to be mixed. Either the lack of a connector or the groove under the switch will cause the switch to not activate and the laser power will not be mixed in.

Regarding mixing of laser treatment energy or light, laser light is delivered to the system via a preferably 50 micron optical fiber or equivalent. Laser light exiting the delivery fiber is preferably collimated using a 16 mm focal length achromatic lens or equivalent. If all safety requirements are met (i.e. laser output compatible fiber inserted and selection switch for laser output activated) a steering mirror reflects the collimated laser light into the center of the illumination axis. This results in the output of the fiber having a cone of white light with a shadow in the center nearly filled with the laser aiming beam (treatment beam during treatment). That is, the laser provides an aiming beam, typically red, when not fully activated for treatment and a treatment beam, typically green, when fully activated. Without the shadow caused by the steering mirror the aiming beam would be entirely washed out or imperceptible except at very low illumination levels.

As described, unique to the present art is an illumination and laser source which heretofore has not be available or utilized. Also unique to the present art is a highly efficient illumination system which utilizes spherical reflectors and associated lenses to capture a maximum light output and also provide a twin path illumination light output from a single lamp source in order to feed fibers of diameter less than 500 microns which are conventionally used for laser treatment only. Further unique to the present art is a laser steering mirror having a solenoid selectability which provides an aiming hole within the illumination path for laser placement. Still further unique to the present art is an illumination arc lamp system having an extremely small point light source which allows for an extremely small illumination focus size or numerical aperture output. Also unique to the present art is an arc lamp mount which precisely places the plasma ball of the arc lamp at the focus center of the optics system. Also unique to the present art is a unique dimming mechanism which moves the focal point of a dimming lens in order to provide dimming without introducing artifacts, chromatic aberrations, or changes of color temperature. Also unique to the present art is a capability of connection with existing conventional laser light sources whereby laser treatment and illumination are both provided at an output of the present art apparatus.

The present art invention also represents a novel apparatus and method for providing the ophthalmic surgeon with graphical photoxicity risk information in a clear and easy to understand manner. In a preferred embodiment, an inexpensive card is removably attached to the control panel of the surgical light source. Preferably, the present art card is attached in close proximity to the light intensity control in order to show the relationship between the output intensity of the light source and the likelihood of photic injury. The graphical representation on the card acts as a guide for adjustment of the output intensity of the source in relationship to an accepted standard, that is such as the "Millennium" from Bausch and Lomb®. In this way the spectral and power characteristics of the various elements involved in delivering light to the eye are integrated into a single and easily manageable variable. This greatly reduces the complexity of judging the best intensity to use in a given situation.

Where provided herein, dimensions, geometrical attributes, and thread sizes are for preferred embodiment informational and enablement purposes. Alternative embodiments may utilize a plurality of variations of the aforesaid without departing from the scope and spirit of the present invention. The art of the present invention may be manufactured from a plurality of materials, including but not limited to metals, plastics, glass, ceramics, or composites.

### BRIEF DESCRIPTION OF THE DRAWINGS

Numerous other objects, features, and advantages of the invention should now become apparent upon a reading of the following detailed description taken in conjunction with the accompanying drawings, in which:

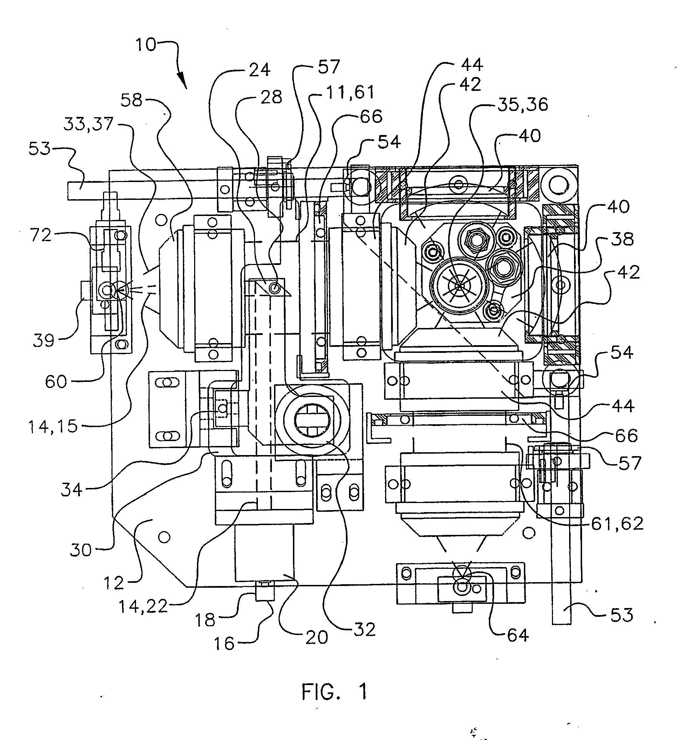

Fig. 1 is a top plan view of a preferred embodiment of the coaxial illuminated laser endoscopic probe and active numerical aperture control apparatus showing illumination and laser light paths without the phototoxicity card, power meter, and ferrule connectors.

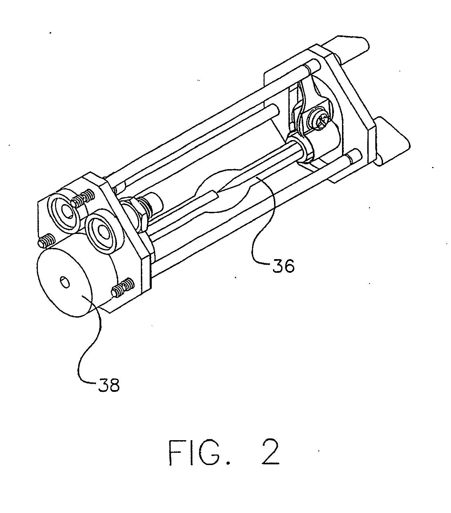

Fig. 2 is a perspective view of the arc lamp source and mount.

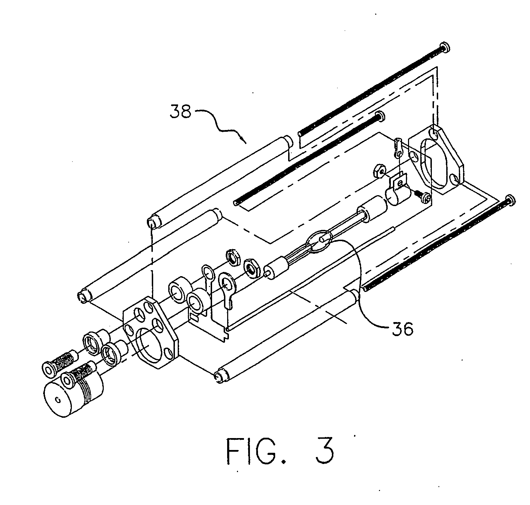

Fig. 3 is an assembly view of the arc lamp source and mount.

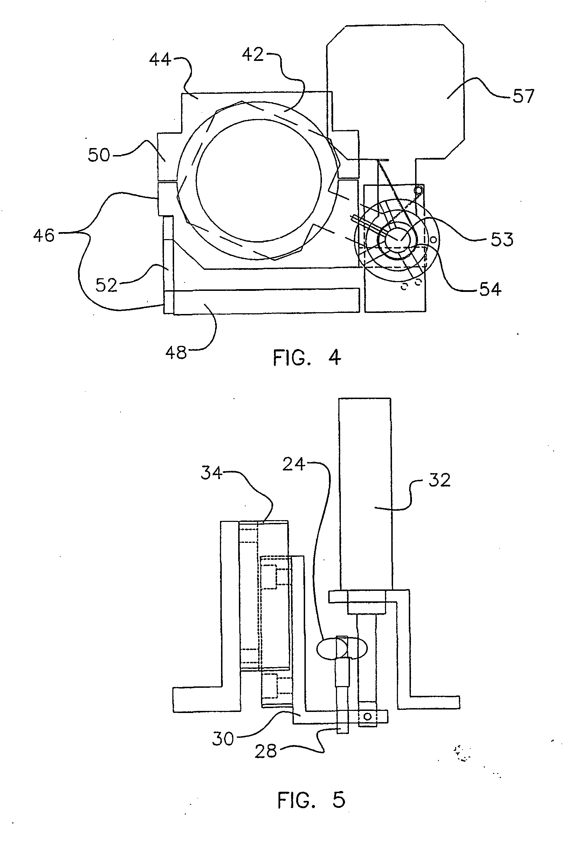

Fig. 4 is a front side plan view of the first lens mount, shaft mounted cam, and shutter with a closed position shutter shown in phantom.
Fig. 5 is a front side plan view of the steering minor, post, bracket, ball slide, and solenoid in a non-energized extended position.

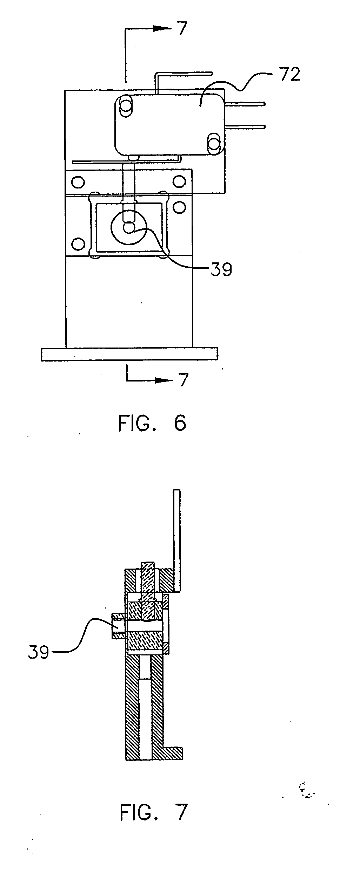

Fig. 6 front side plan view of the first output for laser and illumination light and the switch for sensing the recess in the alignment barrel.
Fig. 7 is a cross sectional view taken along line 7 - 7 of Fig. 6 without the switch body attached.

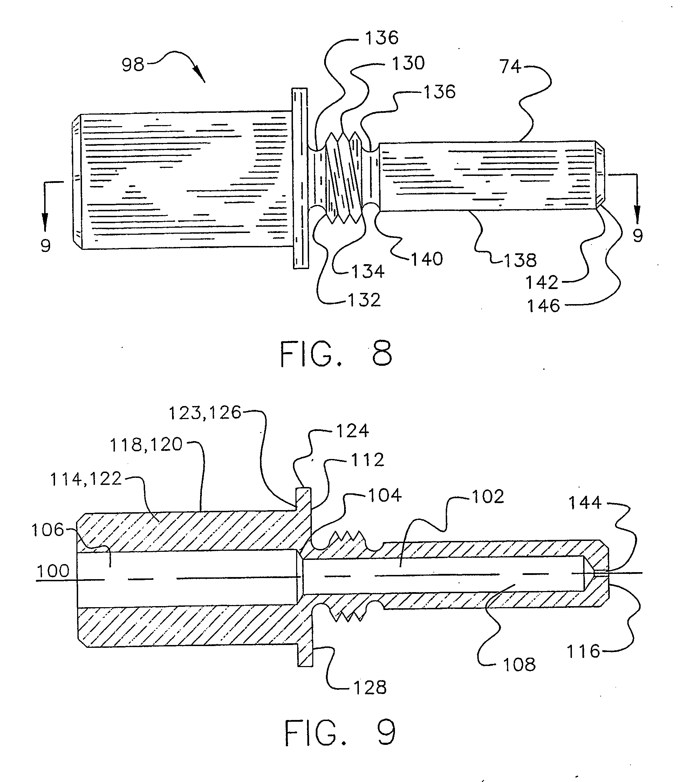

Fig. 8 is a side plan view of the ferrule connector without the recess for preferrably laser and illumination use.
Fig. 9 is a cross sectional view taken along line 9 - 9 of Fig. 8.

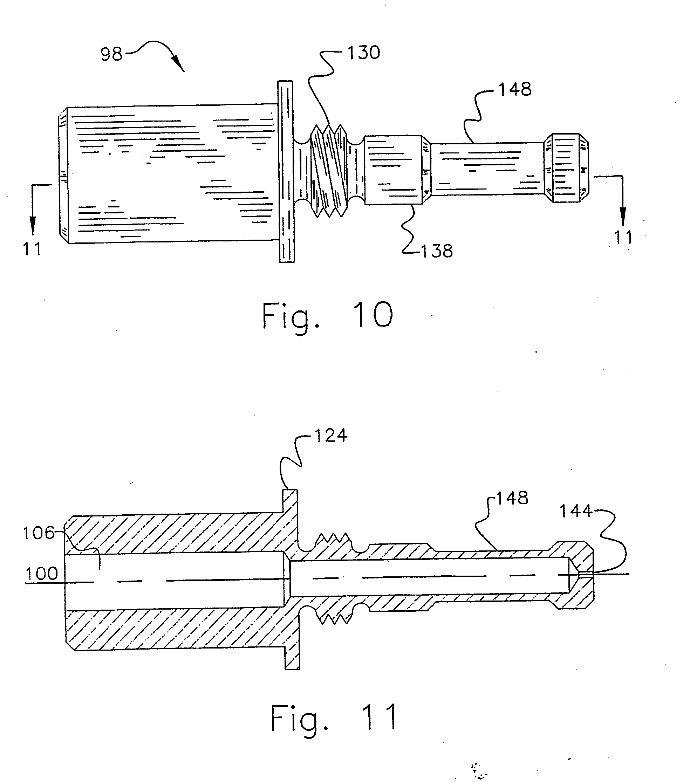

Fig. 10 is a side plan view of the ferrule connector with the recess for preferably illumination use.
Fig. 11 is a cross sectional view taken along line 11 - 11 of Fig. 10.

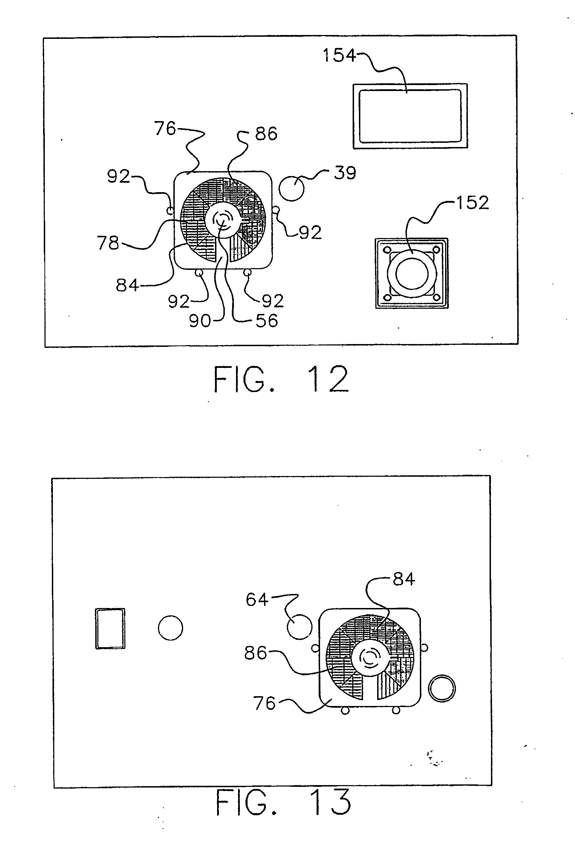

Fig. 12 is a front side plan view of the front panel of the coaxial illuminated laser endoscopic probe and active numerical aperture control apparatus housing showing the first output, illumination level control knob, photoxicity risk card, and laser power meter display and sensor.

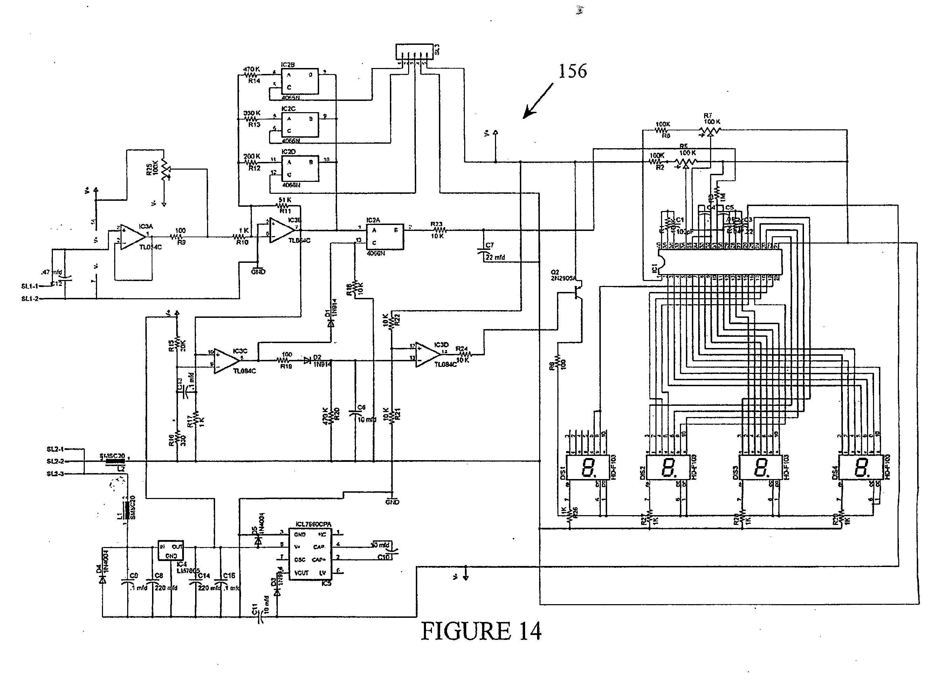

Fig. 13 is a right side plan view of the right panel of the coaxial illuminated laser endoscopic probe and active numerical aperture control apparatus housing showing the second output, illumination level control knob, laser connector, power and laser switches, and photoxicity risk card. Fig.14 is an electronic schematic diagram of the laser power meter circuitry.

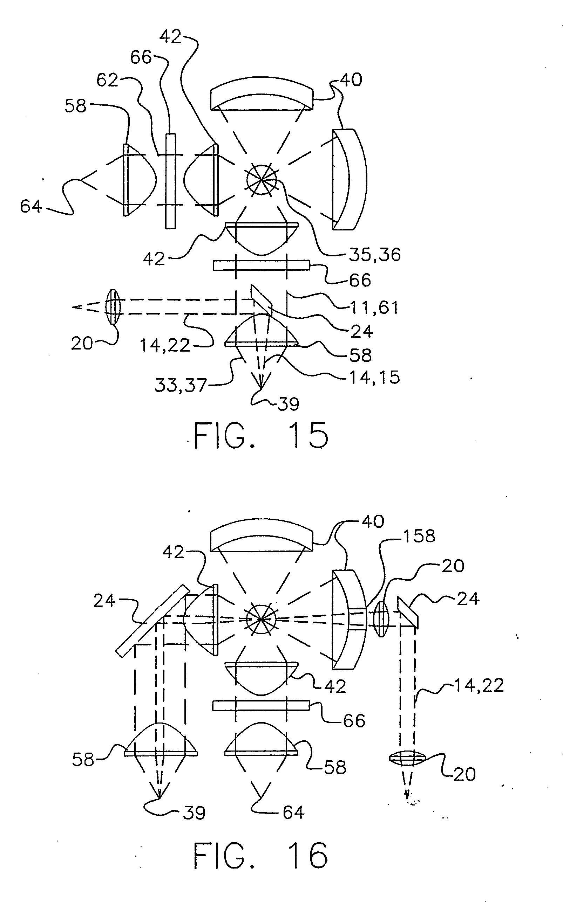

Fig. 15 is an optical schematic diagram of the preferred embodiment of the coaxial illuminated laser endoscopic probe and active numerical aperture control apparatus showing laser and illumination rays, reflectors, mirrors, and lenses.
Fig. 16 is an optical schematic diagram of an alternate embodiment of the coaxial illuminated laser endoscopic probe and active numerical aperture control apparatus showing laser and illumination rays, reflectors, mirrors, and lenses.

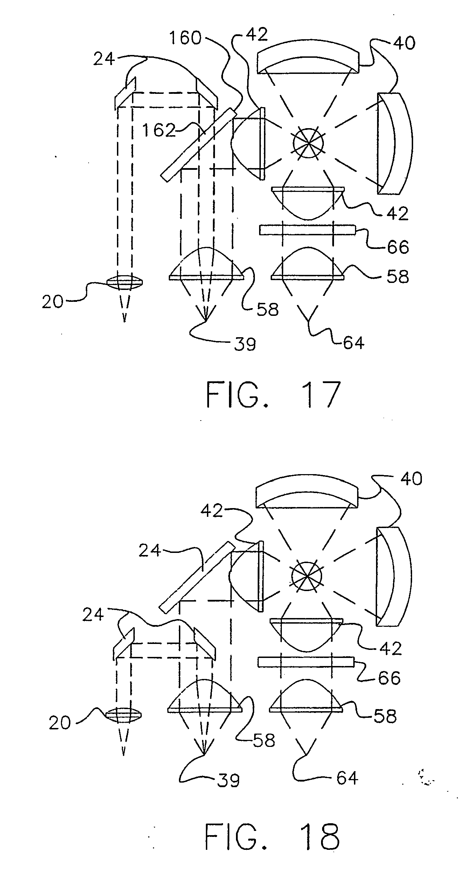

Fig. 17 is an optical schematic diagram of a further alternate embodiment of the coaxial illuminated laser endoscopic probe and active numerical aperture control apparatus showing laser and illumination rays, reflectors, mirrors, and lenses.
Fig. 18 is an optical schematic diagram of another alternate embodiment of the coaxial illuminated laser endoscopic probe and active numerical aperture control apparatus showing laser and illumination rays, reflectors, mirrors, and lenses.

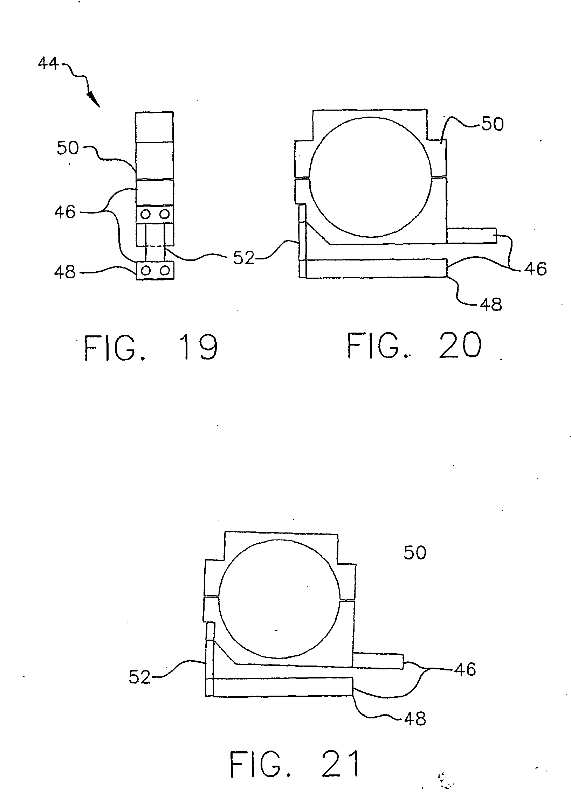

Fig. 19 shows a left side plan view of the first lens mount.
Fig. 20 shows a front side plan view of the first lens mount at a full intensity position
Fig. 21 shows a front side plan view of the first lens mount at a dimmed intensity position.

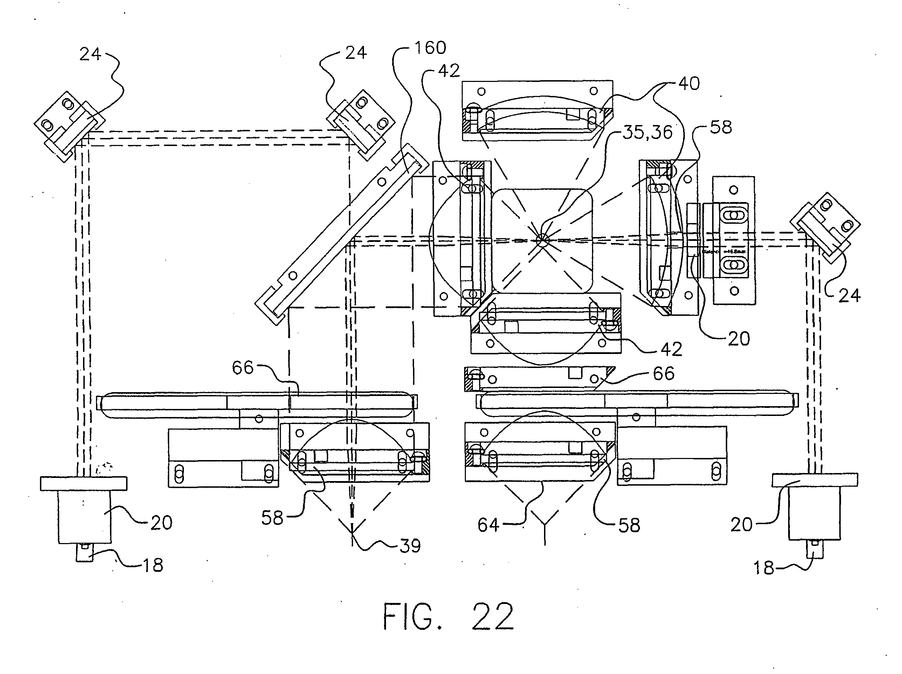

Fig. 22 shows a top plan view of an implementation of the alternate embodiments of the coaxial illuminated laser endoscopic probe and active numerical aperture control apparatus as shown in the optical schematics of Figs. 16 & 17 showing illumination and laser light paths without the phototoxicity card, power meter, and ferrule connectors .

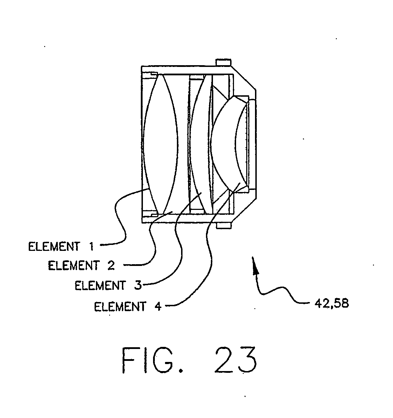

Fig. 23 shows a side plan half cross sectional view of the preferred embodiment of the first and second lenses which correct for color, spherical aberration, and coma and have a back focus 20mm from the apex of the last element and a numerical aperture of .5.

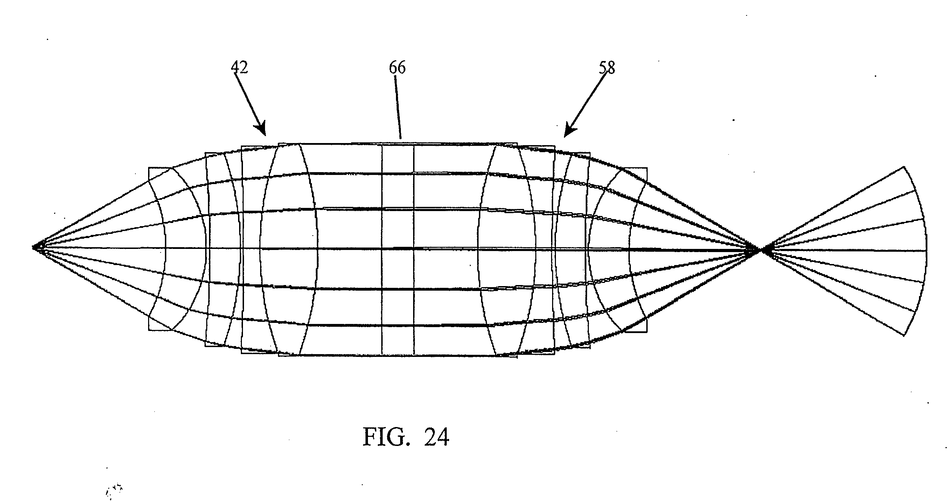

Fig. 24 shows an optical schematic of the first lens set, collimated space, dichroic hot mirror filter, and second lens set with illumination light path rays shown.

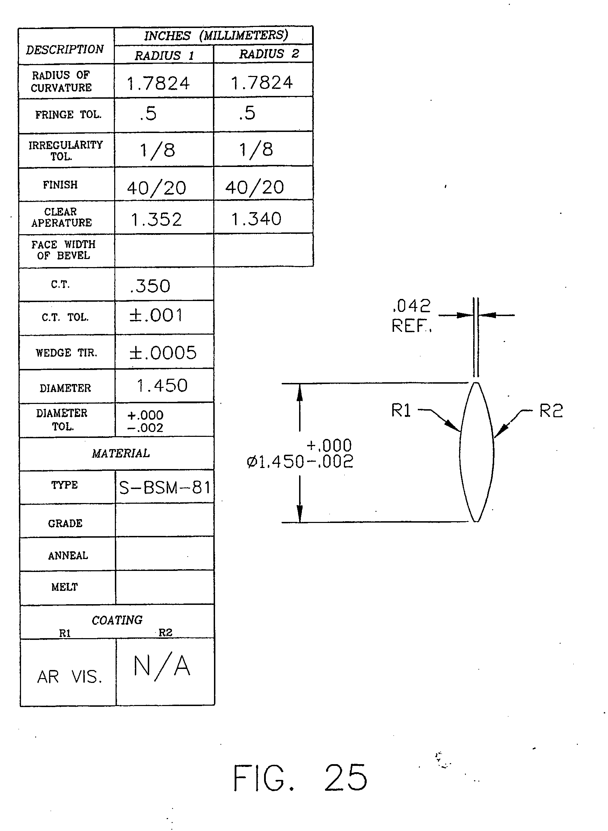

Fig. 25 shows a detailed side plan half cross sectional view with dimensional attributes of the preferred embodiment of element 1 of the lens shown in Fig. 23.

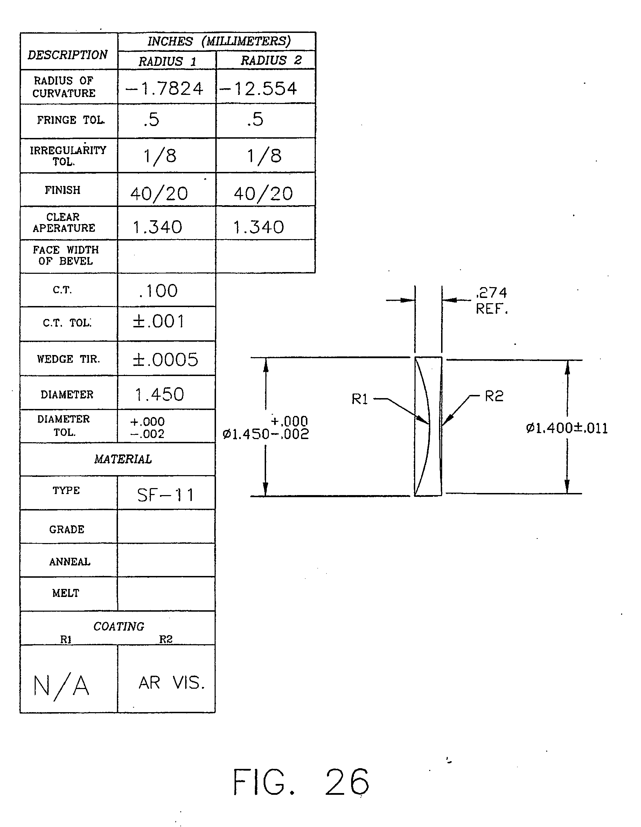

Fig. 26 shows a detailed side plan half cross sectional view with dimensional attributes of the preferred embodiment of element 2 of the lens shown in Fig. 23.

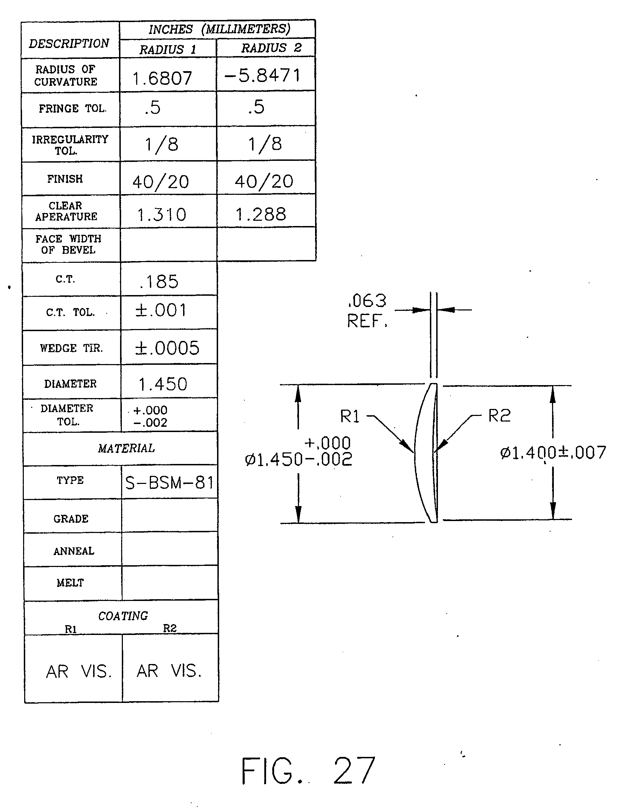

Fig. 27 shows a detailed side plan half cross sectional view with dimensional attributes of the preferred embodiment of element 3 of the lens shown in Fig. 23.

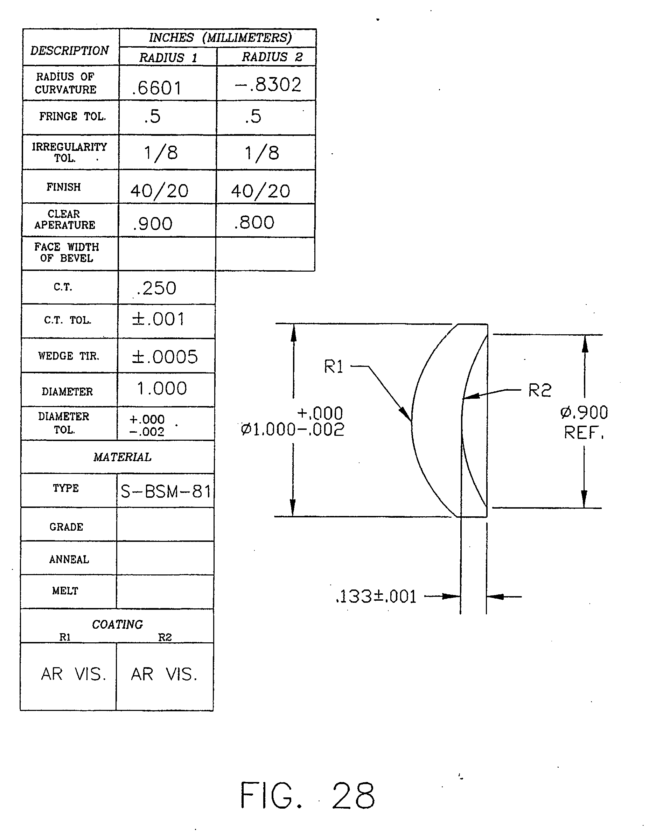

Fig. 28 shows a detailed side plan half cross sectional view with dimensional attributes of the preferred embodiment of element 4 of the lens shown in Fig. 23.

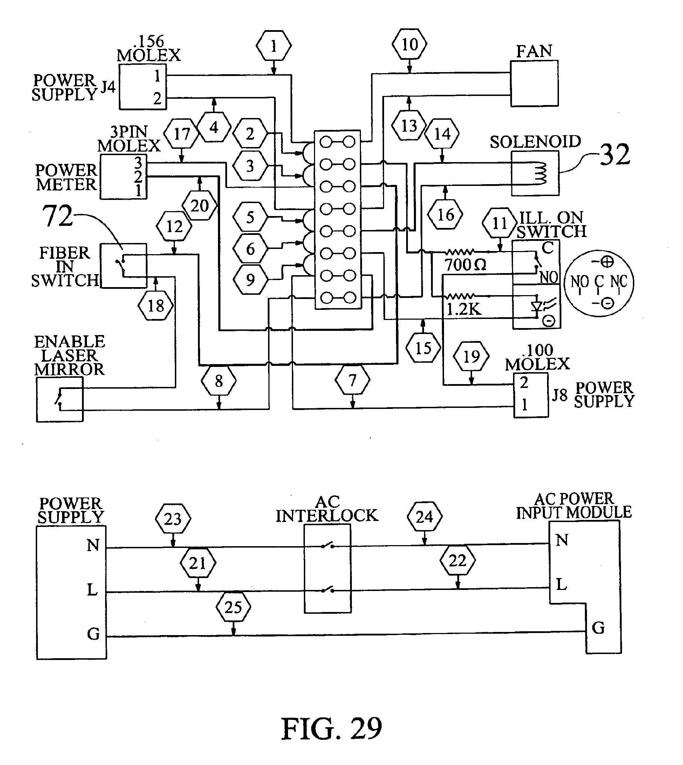

Fig. 29 shows an electrical schematic diagram of the coaxial illuminated laser endoscopic probe and active numerical aperture control apparatus.

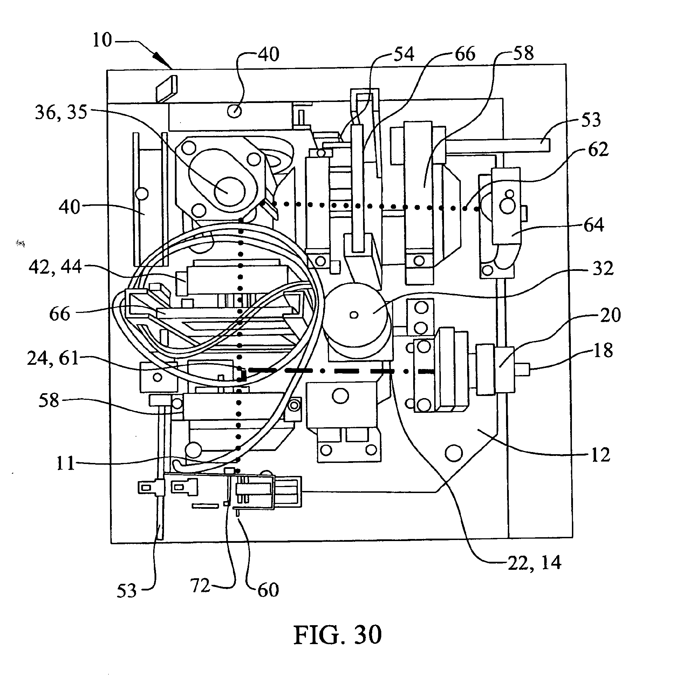

Fig. 30 shows a top perspective view in black and white photographic form of a preferred embodiment of the coaxial illuminated laser endoscopic probe and active numerical aperture control apparatus showing illumination and laser light paths without the phototoxicity card, power meter, and ferrule connectors .

### DETAILED DESCRIPTION

Referring now to the drawings, there is shown in the Figures both preferred and alternate embodiments of the coaxial illuminated laser endoscopic probe and active numerical aperture control apparatus **10** also herein described as an illumination and laser source **10**. There is provided a device **10** for providing non-coherent illumination light **11, 62** and coherent laser treatment light **14** through a single optical fiber **60** of the size typically used for laser treatment only in a safe, effective, and user friendly manner. The apparatus is especially suited for use during ophthalmic surgery.

The present art, in a preferred embodiment, utilizes a 75 watt xenon arc lamp **36** for its high luminance illumination (light density), greater than 6000 °K color temperature, and greater than 95 color rendering index. A unique and useful feature is the very high luminance and small size plasma ball formed on the end of the lamp **36** cathode. If imaged correctly the plasma ball is bright enough to provide the required illumination input to a small fiber such as that used for laser treatment. The xenon arc lamp **36** further provides an extremely small point light source which allows for a smaller output illumination beam **37** diameter. Unique to the present lamp source is a mount 38 which allows for replacement of the lamp **36** and yet retains the location of the plasma ball of said source **36** precisely at a predetennined location within the optical center 35 of the apparatus.

A classic spherical reflector **40** and two lens **42**, **58** light collection layout is utilized rather than other lower part count layouts, such as using an elliptical reflector or a combination of a parabolic reflector and lens. This technique allows maximum collection efficiency with a minimum of geometric aberration. The lamp **36** is located at the geometrical center 35 of the reflector 40 and at the focus (focal point) of the first lens **42**. Light that is incident on the reflector 40 is reflected back to the lamp **36**. This forms an upside down or inverted image of the source 36 coincidental to the source **36**. The first lens **42** collimates light from the source 36 and the upside down or inverted image. The second lens **58** is located coaxial to the first lens 42 and focuses the light at its focal point. The output optical fiber 60 is located at the focal point of the second lens 58. The aforesaid reflectors **40** are preferably spherical rather than parabolic in order to reflect illumination light in the same form as sourced from the arc lamp 36.

Best form lenses **42, 58** (plano convex aspheric, facing each other) are used in the present art. It was discovered that chromatic aberrations, caused by the lenses, gave the output of the optical fiber 39, 60 either a yellow or blue cast. This is not a problem with other ophthalmic sources because the source is many times larger than the output optical fiber. A color corrected "f1" or possibly .5 numerical aperture lens set **42, 58** consisting of four elements was designed to be utilized for each lens. Each of the elements is coated with a MgF (magnesium fluoride) anti-reflective coating to minimize light losses, with other anti-reflective coatings or layers also utilizable. Use of the achromatic lens sets allows a high fidelity image of the illumination source 36 to be focused onto the end of the optical fiber **60, 64**. That is, the multi-element lenses allow for a minimum of chromatic aberration. The aforesaid four element lens set is shown and specifically described in the Figures.

An additional separate illumination path **62** is possible with the present art. A 0.5 system numerical aperture or "f 1" lens is the greatest practical because of limitations to the numerical aperture of available optical fibers. This equates to 60 degrees full angle. When the spherical reflector 40 is considered, an additional 60 degrees is provided from the total of 360 degrees available. Consideration of the vertical rotation around the source **36** is impractical because of shadows caused by the lamp 36 electrodes. A total of 240 degrees of horizontal rotation around the lamp 36 are left unaccounted for. Allowing for optics mounts **44** does account for some additional amount. However, at least half of the illumination output is available. This leaves room for a second light path 62 located orthogonal to the first path **11** along with the second fiber output 64. No other conventional illumination light source incorporates multiple light paths from a single lamp, that is two independent collection systems for illumination light. The independent nature of the two paths **11, 62** allow different filtering and intensity control settings to the two outputs **39, 41**.

Output dimming of the present art illumination system is accomplished by steering the first (collimating) or penultimate lens **42** in a fashion that does not change the lens **42** numerical aperture or introduce shadow artifacts into the beam **37**. The lens set mount **44** has two halves 46 and a flat spring **52.** The first part, **48** is attached to the optics bench **12**, the second part 50 holds the lens set 42, and the spring 52 connects the two **48, 50** together on one side. Pressure on the lens mount second part 50 causes the spring 52 to deflect and the lens **42** to move in a direction generally perpendicular to the optical axis. This results in motion or movement of the image across the face of the optical fiber **60, 64** whereby the peak illumination of the beam 37 is not centered on the optical fiber **60, 64** face during dimming. Due to the aforesaid, the reduction of the output light from the fiber **60, 64** without affecting the color (i.e. color temperature) or aperture of the output is achieved. In a preferred embodiment a shaft mounted cam **54** applies the pressure to the lens mount second part **50** and spring **52**. A control knob 56 is attached to the other end of the shaft **53** and allows the user to select the desired illumination level by rotating the knob **56**. This method is capable of providing at least 95% reduction in output illumination intensity. In a preferred embodiment, a shutter **57** is mounted upon the shaft **53** and is rotated across the illumination beam 37 in order to fully attenuate the output illumination intensity upon full rotation of said knob 56. Alternative embodiments may utilize other methods, including but not limited to electric or electronic drives, to rotate said shaft **53** instead of said knob **56**.

A dichroic "hot" mirror filter **66** is placed in the collimated space **61** between the illumination lenses **42, 58**. This provides both UV and IR filtering of the light. Brackets are attached to the hot mirror **66** mount to provide a means for additional user selectable filters. Positioning of the filters is critical because this is the only area where the light **11** is generally normal to the filter surface. Location of the filter 66 on the other sides of the lenses would cause the light to have many undesirable incidence angles (between 0 and 30 degrees). Variation in the incidence angle causes dichroic reflectors or filters to have a shift in their affect. If absorption filters are used, placement outside the collimated space **61** will cause an increase in reflective losses and heating problems.

The output optical fiber connector **98** is uniquely configured to provide the precise positioning required while reducing cost. A precise connector or mating end **116** is combined with an integral retention thread **130** to reduce parts cost and assembly time. An optional groove or recess **148** is placed on a second version of the connector to provide for sensing the difference between illumination only and laser compatible output fibers. Placement of a smooth diameter connector **74** into the output activates a switch **72** which will allow the laser power to be mixed. Either the lack of a connector 98 or the groove or recess **148** under the switch **72** will cause the switch **72** to not activate and the laser power will not be mixed in.

Regarding mixing of laser treatment energy or light **14**, laser light 14 is delivered to the system via a preferably **50** micron optical fiber **16** or equivalent. The connector **18** on the laser end is configured to be compatible with the laser and to provide the necessary interface to signal to the laser that a fiber is connected. The laser and light source **10** end preferably uses an SMA 905 connector or equivalent to allow repeatable connections of the laser delivery fiber **16**. Laser light **14** exiting the delivery fiber is preferably collimated using a 16 mm focal length achromatic lens or equivalent **20,** i.e. laser collimating lens, which can also be utilized to focus the collimated laser beam **22**. The position of the fiber **16** is adjusted to be at the focal point of the lens **20.** The input laser connector 18 and collimating lens **20** are located so that the collimated beam **22** is orthogonal to and intersects the center of the illumination axis 11 between the illumination lens sets **42, 58** (the collimated area for illumination light). If all safety requirements are met (i.e. laser output compatible fiber inserted and selection switch for laser output activated) a steering mirror **24** reflects the collimated laser light 22 into the center of the illumination axis 11. The steering mirror **24** is a first surface plano that is positioned at 45 degrees to the laser light 14 and is located in the center of the illumination axis 11 (when laser mode is active). A unique aspect of the present invention is that the thickness of the mirror 24 is shaped to appear as a circle when viewed along the illumination axis. Due to the 45 degree surface orientation, the shaping causes the mirror **24** surface to appear elliptical when viewed from a normal angle. The size of the mirror **24** is chosen to be minimally larger that the collimated laser beam **22**. Placement of the steering mirror **24** in the center of the illumination axis 11 causes the light rays that would normally be there to be blocked and a shadow to appear in the center of the output light cone. The second illumination lens **58** focuses the laser light **14** reflected by the steering mirror **24** onto the end of the output fiber **60, 64**. Because the length of the output optical fiber strand is relatively short, the incidence angle of light entering the input end is very nearly the same angle on the output end. This results in the output of the fiber strand having a cone of white light with a shadow in the center nearly filled with the laser aiming beam (treatment beam during treatment). That is, the laser provides an aiming beam, typically red, when not fully activated for treatment and a treatment beam, typically green, when fully activated. Without the shadow caused by the steering mirror **24** the aiming beam would be entirely washed out or imperceptible except at very low illumination levels.

Alternate embodiments may utilize more than one steering mirror **24** or place the steering mirror **24** outside of the illumination axis or illumination light path **11** and direct the laser light **14** through an aperture **158** in said spherical reflector **40** and thereafter through the arc lamp 36 plasma ball or through a dichroic reflector **160** or a reflector having an aperture 162. All of the aforesaid alternate embodiments place the laser light **14** within the collimated space 61 and utilize the second lens 58 for focus upon the output optical fiber **60**. Moreover, all of the aforesaid alternate embodiments provide for a second light path **62** output as seen in the Figures.

The laser steering mirror **24** is mechanically mounted on a thin post **28** that holds it in place while minimizing the loss of illumination light **11**. The post **28** is mechanically connected to a bracket **30** which is connected to a solenoid **32**. The solenoid **32** causes the bracket **30** and also the steering mirror **24** to move into one of two positions. Position one is outside the collimated illumination and laser light. This position is used for no laser delivery and allows the illumination path to operate unaffected. Position two is with the steering mirror **24** located to reflect the laser light into the illumination path **11**. Motion of the solenoid **32** and bracket **30** are controlled by a precision ball slide **34**. Use of the slide **34** insures repeatable positioning of the mirror **24**.

As described, unique to the present art is a coaxial laser and illumination path apparatus 10 which heretofore has not be available or utilized. Also unique to the present art is a highly efficient illumination system which utilizes spherical reflectors **40** and associated lenses **42, 58** to capture a maximum light output and also provide a twin path illumination light output from a single lamp source in order to feed fibers of diameter less than 500 microns. Further unique to the present art is a laser or steering mirror **24** having a solenoid **32** selectability which provides an aiming hole within the illumination path **11** for laser placement. Still further unique to the present art is an illumination arc lamp **36** system having an extremely small point light source 36 which allows for an extremely small illumination focus size or numerical aperture output. Also unique to the present art is an arc lamp **36** mount **38** which precisely and interchangeably places the plasma ball of the arc lamp **36** at the focus or optical center **35** of the optics system. Also unique to the present art is a unique dimming mechanism which moves the focal point of an output dimming or first lens **42** in order to provide dimming without introducing artifacts, chromatic aberrations, or change of color temperature. Also unique to the present art is a capability of connection with existing conventional laser light sources whereby laser treatment and illumination are both provided at an output of the present art apparatus **10**. The optical system of the present apparatus **10** is uniquely capable of accepting the input cone angles of the illumination **33** and laser light **15** placed at the output optical fiber 60 and substantially reproducing said cone angles at the output of the optical fiber, typically where the endoscopic probe is located, with any aberrations caused by the optical fiber itself.

Further alternate embodiments of the present art apparatus **10** may utilize parabolic reflectors instead of spherical reflectors in order to collimate the illumination source **36**. This technique would eliminate the need for the first collimating lens **42** and allow transmission of the laser beam **22** through an aperture within the parabolic reflector or via a steering mirror **24** within the collimated space **61**. Still further alternate embodiments may utilize an elliptical reflector having two focal points whereby the illumination source **36** is placed at the first focal point and the output fiber **60** is placed at the second focal point with the laser beam **22** introduced through an aperture within the elliptical reflector or via a steering mirror **24** between the illumination source **36** and the output fiber **60**. This latter alternate embodiment requires focusing the laser beam **22** onto the output fiber **60** via a lens placed within the laser beam path 22 prior to the output fiber 60 yet allows elimination of both the first collimating lens **42** and the second focusing lens **58**.

Some of the variables which determine the phototoxicity risk level during vitreoretinal surgery include the spectral and power characteristics of the light source used, the type and size of the endoilluminator probe, the length or duration ofthe surgical procedure, and the area (size) of the illuminated tissues. In each case the surgeon must make a risk-benefit judgement about the intensity of light to be used. Use of insufficient intensity may result in inadequate visualization and adverse effects more serious than a retinal photic injury. Currently, the calculation of the exposure time required to reach a point of injury is a tedious chore involving the numerical integration of the spectral power density function of the light source **36** with a hazard function (see ISO 15752), and specific knowledge of the surgical illumination area and endoilluminator characteristics.

The present art invention further represents a novel apparatus and method for providing the ophthalmic surgeon with graphical photoxicity risk information in a clear and easy to understand manner. In a preferred embodiment, an inexpensive photoxicity risk card **76** is removably attached to the control panel of the surgical illumination and laser light source 10. Preferably, the present art card 76 is attached in close proximity to the light intensity control knob **56** in order to show the relationship between the output intensity of the light source and the likelihood of photic injury. The card **76** is preferably included with each endoilluminator instrument, i.e. optical fiber, that is calibrated to represent the phototoxic performance of that instrument type when used with a particular type of light source. The graphical representation **78** on the card **76** acts as a guide for adjustment of the output intensity of the source **10** in relationship to an accepted standard, that is such as the "Millennium" from Bausch and Lombe®. In this way the spectral and power characteristics of the various elements involved in delivering light to the eye are integrated into a single and easily manageable variable. This greatly reduces the complexity of judging the best intensity to use in a given situation. Alternative embodiment graphical representations **78** could present other information regarding the light output such as lumen output (a unit that is weighted by the photopic response of the eye). Other representations could present threshold information when used with special dyes or colored light filters.

A preferred embodiment of the invention comprises a card 76 that is die-cut from white chipboard stock that is approximately the weight of a business card. The shape of the card 76 is generally square with a slot **90** removed from one side to enable the card **76** to be placed behind the intensity control knob **56** of the illumination and laser source **10** while providing clearance for the control shaft **53** which is turned by said knob **56**. In a preferred embodiment, four location pins **92** are attached to the front panel of the illumination and laser source 10 enclosure. The pins **92** provide boundaries for card 76 location and tend to inhibit rotation of the card 76 with the control knob.

In a preferred embodiment, onto the face of the card is printed a circular shaped scale **84** that has different color bands **86** representing the phototoxicity risk at a given intensity level, for example green, yellow, and red. The control knob **56** has an indication line that points to the current output intensity level and concurrent phototoxicity risk associated with the probe being used. Unique to the present art is the ability of the card **76** to indicate output intensity at the optical fiber output. The card **76** is meant to be disposed of after a single use and replaced with a new one provided with each optical fiber instrument. In this manner the output of the light source 10 is recalibrated each time it is used. The calibrated unit type-may vary with different instrument styles to provide the surgeon with the most pertinent information possible.

As aforesaid the card **76** provides a known point of reference relative to the prior art illumination devices. For example, if the surgeon maintains the knob **56** indicator line within the green color band, he or she will understand that the light intensity output is within the safe intensity of the prior art illuminators such as the "Millennium" from Bausch and Lomb®. This control phenomena is especially useful when utilizing more powerful illumination sources **10** such as described herein. That is, the surgeon must have a prior art point of reference when utilizing more powerful and modem illumination systems such as the present art. The art of the present invention may further provide several bands which do not provide a reference to the prior art but instead indicate phototoxicity levels or light intensity levels directly to the surgeon.

The manufacturer of the optical fiber can provide a phototoxicity risk card 76 which accounts for attenuation and spectral absorption within the optical fiber provided with said card 76. Thus for example, if an optical fiber is highly attenuating, the card may indicate that the surgeon must turn the intensity control knob **56** to a higher level in order to obtain an equivalency to one or more of the aforesaid prior art illuminators or to achieve a desired photo- illumination output.

A ferrule or connector **98** has an internal bore **102,** preferably stepped **104,** which is substantially parallel with the lengthwise axis **100** of the ferrule body **98.** The aforesaid bore **102** allows for placement and bonding or potting of an optical fiber within and through said ferrule body **98.** Externally, said ferrule body **98** is also stepped **112, 148** in a unique form in order to optimally function as described herein.

In a preferred embodiment, the ferrule body **98** has an external end **114** and a mating end **116** and externally comprises a substantially cylindrical head **118** of a first diameter **120** having a first end **122** and a second end **123,** said first end **122** co-located with said external end **114.** Said ferrule body **98** further externally comprises a lip **124** of greater diameter than said head **118** and having a first side **126** and a second side **128** with said first side **126** mounted with said second end **123** of said head **118.** A threaded portion **130** of preferably smaller diameter than said head **118** is attached with and extends from said lip **124** second side **128.** In a preferred embodiment, said threaded portion **130** first comprises an 8-32 UNC thread with a first end **132** and second end **134,** said first end **132** connected with said second side **128** of said lip **124.** Also in a preferred embodiment, said threaded portion **130** has a groove **136** of approximately 0.76 mm (.030 inch) at said first end **132** with approximately 2.29 mm (.090 inch) of said thread **130** thereafter following and another approximately 0.76 mm (.030 inch) groove **136** following said thread **130** at said second end **134.** Externally the ferrule body **98** also has an alignment barrel **138** having a first **140** and second **142** end following said threaded portion **130,** said first end **140** attached with said threaded portion **130.** The second end **142** of said alignment barrel **138** is co-located with said mating end **116** of said ferrule body **98.** Also, said second end **142** of said alignment barrel **138** contains an orifice **144** of substantially equivalent or slightly greater diameter as the optical fiber mounted within said stepped bore **102.** Said orifice **144** is interconnected with said internal stepped bore **102.** In an embodiment, said orifice is approximately 0.28 mm (.011 inch) in diameter and 6.35 mm (.025 inch) in length. Also in a preferred embodiment, said alignment barrel **138** has a chamfer **146** at the circumference of said second end **142.** Preferably said chamfer **146** is of approximately 45 degree angle and 0.38 mm (.015 inch) in length. Alternative embodiments may utilize chamfers of different angles or shapes or forego use of a chamfer altogether.

The alignment barrel **138** is uniquely shaped within the embodiments to indicate whether laser light or illumination light should be applied to the optical fiber. In a preferred embodiment of the laser ferrule, the alignment barrel is of uniform diameter, approximately 3 mm (.118 inch) diameter, which indicates to the source 36 that laser light or energy is desired. In a first alternative embodiment or illumination ferrule, the alignment barrel contains a recess **148** located approximately 1.9 mm (.075 inch) from said barrel **138** second end **142** and extending approximately .268 inch from said second end **142.** When utilized, the illumination and laser source **10** detects this recess and determines that illumination light and not laser light is desired. Further alternative embodiments may utilize the aforesaid recess **148** embodiment for laser light and the uniform barrel diameter for illumination light.

Internally said stepped bore **102** first comprises a first larger bore substantially within said head portion which is of approximately 2.49 mm (.098 inch) diameter and extends substantially the length of said head. A second intermediate bore of approximately 1.6 mm (.063 inch) diameter extends from said first larger bore to said orifice **144** within said threaded portion **130** and said alignment barrel **138.** Also in a preferred embodiment, the orifice **144** length is approximately 0.635 mm (.025 inch). Alternative embodiments may utilize first and second bores and orifices having a plurality of diameter and length sizes provided that the diameter portions are smaller than the ferrule external portions within which each is located.

When assembled with an optical fiber, the optical fiber extends through said bore **102** and orifice **144** and terminates substantially flush with said ferrule body **98** mating end **116** or second end **142** of said alignment barrel **138.** Preferably said optical fiber is held within said bore **102** via potting or adhesive compounds surrounding said fiber and attaching with said bore **102** of the ferrule **98.**

In a preferred embodiment, the external head **118** diameter is approximately 5.9 mm (.234 inch) with a length of approximately 9.5 mm (.375 inch). The lip **124** external diameter is approximately 7.9 mm (.312 inch) with a thickness of approximately 0.64 mm (.025 inch). Also, said alignment barrel **138** is approximately 3 mm (.118 inch) in diameter and 9.7 mm (.380 inch) in length.

Where provided, dimensions, geometrical attributes, and thread sizes are for preferred embodiment informational and enablement purposes. Alternative embodiments may utilize a plurality of variations of the aforesaid without departing from the scope and spirit of the present invention. This is especially true as relating to said head **118,** lip **124,** and threaded portion **130.** Said lip **124** may be integrated as part of the head **118** or removed completely. Also, the position, location, and type of threaded portion **130** may vary. Said threaded portion **130** may not utilize said grooves **136,** utilize grooves of a shorter or longer length, or have said head **118** and lip **124** diameters sized substantially the same as or smaller than the outside diameter of said threads **130.** The ferrule connector may be manufactured from a plurality of materials, including but not limited to metals, plastics, ceramics, or composites.

A laser power meter has a sensor **152,** a power display **154,** and associated control circuitry **156.** The power meter allows a surgeon to place the endoscopic fiber optic probe onto said sensor **152,** energize the laser through the illumination and laser source **10** and measure the laser power output as seen on said display **154.** Inclusion of the aforesaid is especially useful due to variations in optical fibers or to account for attenuation through the illumination and laser source **10.** By utilizing the power meter, the surgeon has complete knowledge of the laser power transmitted to the surgical site. Alternative embodiments may utilize said power meter for measurement of the output illumination **37** intensity as well as the laser light **14** power.

In operation, the surgeon connects a laser light source via optical fiber to the input laser connector **18** on the apparatus **10.** The surgeon thereafter connects a ferrule connector **98** with an integral optical fiber connected with an endoscopic probe at the first output **39** or for illumination only at said second output **64.** If said ferrule connector **98** at said first output **39** does not have the afore described recess **148,** the apparatus **10** will allow the steering mirror **24** to position within the illumination light path **11** and further allow transmission of laser light. If the surgeon desires to measure laser power output, he or she places the output end of the endoscopic probe onto said sensor **152** and upon full laser activation, reads the laser power output on the display **154.** If the apparatus **10** is powered, the surgeon proceeds to illuminate the tissues of concern with a cone of white illumination light having a shadow where the laser beam will be placed and a typically red laser aiming beam within said shadow. Upon full activation of laser power, a typically green treatment laser beam replaces said typically red aiming beam to treat the tissues of concern. All of the aforesaid illumination and treatment may be achieved with a single incision and through a single optical fiber of smaller diameter than prior art sources.

Those skilled in the art will appreciate that a coaxial illuminated laser endoscopic probe and active numerical aperture control apparatus **10** (illumination and laser source) and method of use such has been shown and described. The apparatus and method of use allows for simultaneous transmission of illumination and laser treatment light through a single optical fiber of a size which is typically utilized for laser treatment light only. The apparatus and method further provides control of the angular light output from the endoscopic probe attached with said optical fiber. The apparatus also provides for distinct and separate illumination without utilization of the treatment laser while providing complete intensity control of said illumination. Those skilled in the art will appreciate that a medical light intensity phototoxicity control or risk card **76** has also been shown and described for use. Said phototoxicity risk card **76** is especially useful for quick and easy determination of illumination intensity output from a specific type of optical fiber or higher power source. Those skilled in the art will appreciate that a photon illumination and laser ferrule connector **98** has also been shown and described. Said ferrule **98** is especially useful for quick and positive connection of an optical fiber to a laser or illumination source **10** as herein described and further allows said source **10** to distinguish the optical fiber type or use, that is for illumination or medical laser application. The present art device is useful during surgery and especially ophthalmic surgery. Also, those skilled in the art will appreciate the integral inclusion of a laser optical fiber output power meter.

Having described the invention in detail, those skilled in the art will appreciate that modifications may be made of the invention. Therefore, it is not intended that the scope of the invention be limited to the specific embodiments illustrated and described. Rather it is intended that the scope of this invention be determined by the appended claims and their equivalents.

## Claims

1. An illumination and laser source comprising
an illumination source (36) having an output of surgically useful visible broad spectrum illumination light;
a first lens (42) adapted to collimate a portion of the illumination light into a collimated illumination light path (11); and
a laser light source having an output of laser light (14), the laser light (14) being collimated to form a collimated laser beam (22); and
a second lens (58) adapted to focus the collimated illumination light (11) and the collimated laser beam (22) onto a single optical fiber (60); wherein
the illumination source (36) has an illumination focus spot of a size sufficiently small to couple the illumination light into the single output optical fiber (60),
the collimated laser beam (22) is positioned to be within the collimated illumination light path (11),
**characterized in that**
the output optical fiber (60) has a diameter of 500 µm or less, and
a dimming mechanism is provided for dimming the illumination light at the output of the optical fiber (60).

2. The illumination and laser source as set forth in claim 1 further comprising
a laser collimating lens (20) adapted to collimate said laser light (14); and
a steering mirror (24) within an axis of said collimated illumination light path and reflecting said collimated laser beam (22) whereby said collimated laser beam (22) is within said collimated illumination light path (11).

3. The illumination and laser source as set forth in claim 2 wherein
said collimated laser beam (22) within said collimated illumination light (11) is substantially parallel with said illumination light (11).

4. The illumination and laser source as set forth in claim 1 further comprising
one or more spherical reflectors (40) having a geometrical centre (35); and
said illumination source (36) located at said geometrical centre (35) and at a focal point of said first lens (42).

5. The illumination and laser source as set forth in claim 2 further comprising
one or more spherical reflectors (40) having a geometrical centre (35); and
said illumination source (36) located at said geometrical centre (35) and at a focal point of said first lens (42).

6. The illumination and laser source as set forth in claim 1 further comprising
a dimming mechanism comprising a mount (44) on said first lens (42) adapted to steer said first lens (42) in a direction substantially perpendicular to an optical axis of said collimated illumination light path whereby a lens numerical aperture of said first lens (42) is not substantially changed.

7. The illumination and laser source as set forth in claim 6 said dimming mechanism mount (44) further comprising
a first part (48) attached with an optics bench (12) and a second part (50) holding said first lens (42), said first part and second part (48, 50) attached with a spring (52) whereby a pressure on said second part (50) causes said spring (52) to deflect and said first lens (42) to move.

8. The illumination and laser source as set forth in claim 7 said dimming mechanism mount further comprising
a cam (54) adapted to apply said pressure to said second part (50).

9. The illumination source as set forth in claim 1, wherein
said illumination source (36) comprises an arc lamp having a mount which allows for replacement of said arc lamp and yet retains a location of a plasma ball of said illumination source precisely at a predetermined location within an optical centre.

10. The illumination source as set forth in claim 1,
wherein said first lens (42) is adapted to steer a portion of said illumination light into a collimated illumination light path, said first lens (42) steering providing a dimming of said illumination light.

11. An illumination and laser source as set forth in claim 1, further comprising
a connector (98) mounted with said optical fiber (60) and adapted to indicate whether said laser light (14) or said illumination light (11) or both should transmit through said optical fiber (60); and
a phototoxicity risk card (76) adapted to provide a representation of illumination risks during dimming,
wherein said first lens (42) is adapted to steer a portion of said illumination light into a collimated illumination light path, said first lens (42) steering providing a dimming of said illumination light.

12. The illumination and laser source as set forth in claim 1 further comprising
a power meter (150) having a sensor (152) to receive a light power, a display (154) to indicate a value of said light power, and a control circuitry (156).

13. The illumination and laser source as set forth in claim 2 wherein
said steering mirror (24) provides a shadow which does not wash out said laser beam within said collimated illumination light path (11) where said collimated laser beam (22) is placed and an output of said optical fibre (60) is adapted to illuminate one or more tissues of concern and said shadow appears on said tissues where said laser beam (22) will be placed for treatment.

## Patentansprüche

1. Beleuchtungs- und Laserquelle mit
einer Beleuchtungsquelle (36), die einen Ausgang von chirurgisch verwendbarem sichtbarem Breitband-Beleuchtungslicht hat,
einer ersten Linse (42), die dazu ausgelegt ist, einen Abschnitt des Beleuchtungslichts in einen kollimierten Beleuchtungslichtpfad (11) zu kollimieren,
einer Laserlichtquelle, die einen Ausgang von Laserlicht (14) hat, wobei das Laserlicht (14) zur Bildung eines kollimierten Laserstrahls (22) kollimiert ist,
einer zweiten Linse (58), die dazu ausgelegt ist, das kollimierte Beleuchtungslicht (11) und den kollimierten Laserstrahl (22) auf eine einzelne optische Faser (60) zu fokussieren, wobei
die Beleuchtungsquelle (36) einen Beleuchtungsfokuspunkt in einer Größe hat, die ausreichend klein ist, um das Beleuchtuhgslicht in die einzelne optische Ausgangsfaser (60) zu koppeln,
der kollimierte Laserstrahl (22) derart positioniert ist, dass er in dem kollimierten Beleuchtungslichtpfad (11) ist,
**dadurch gekennzeichnet, dass**
die optische Ausgangsfaser (60) einen Durchmesser von 500 µm oder weniger hat und
ein Dimmmechanismus zum Dimmen des Beleuchtungslichts an dem Ausgang der optischen Faser (60) vorgesehen ist.

2. Beleuchtungs- und Laserquelle nach Anspruch 1, die weiterhin
eine Laserkollimationslinse (20), die dazu ausgelegt ist, das Laserlicht (14) zu kollimieren, und
einen den kollimierten Laserstrahl (22) reflektierenden Lenkspiegel (24) in einer Achse des kollimierten Beleuchtungslichtpfads aufweist, wobei der kollimierte Laserstrahl (22) in dem kollimierten Beleuchtungslichtpfad (11) ist.

3. Beleuchtungs- und Laserquelle nach Anspruch 2, wobei der kollimierte Laserstrahl (22) in dem kollimierten Beleuchtungslicht (11) im Wesentlichen parallel zu dem Beleuchtungslicht (11) ist.

4. Beleuchtungs- und Laserquelle nach Anspruch 1, die weiterhin einen oder mehrere Kugelreflektoren (40) mit einem geometrischen Zentrum (35) und die Beleuchtungsquelle (36) aufweist, die an dem geometrischen Zentrum (35) und an einem Fokuspunkt der ersten Linse (42) angeordnet ist.

5. Beleuchtungs- und Laserquelle nach Anspruch 2, die weiterhin einen oder mehrere Kugelreflektoren (40) mit einem geometrischen Zentrum (35) und die Beleuchtungsquelle (36) aufweist, die an dem geometrischen Zentrum (35) und an einem Fokuspunkt der ersten Linse (42) angeordnet ist.

6. Beleuchtungs- und Laserquelle nach Anspruch 1, die weiterhin einen Dimmmechanismus mit einer Halterung (44) auf der ersten Linse (42) aufweist, die dazu ausgelegt ist, die erste Linse (42) in eine Richtung zu steuern, die im Wesentlichen senkrecht zu einer optischen Achse des kollimierten Beleuchtungslichtpfads ist, wobei eine numerische Linsenapertur der ersten Linse (42) im Wesentlichen nicht geändert wird.

7. Beleuchtungs- und Laserquelle nach Anspruch 6, wobei die Dimmmechanismushalterung (44) weiterhin einen ersten Teil (48), der an einer Optikbank (12) befestigt ist, und einen zweiten Teil (50) aufweist, der die erste Linse (42) hält, wobei der erste und der zweite Teil (48, 50) mit einer Feder (52) befestigt sind, wobei ein Drücken auf den zweiten Teil (50) ein Biegen der Feder (52) und eine Bewegung der Linse (42) bewirkt.

8. Beleuchtungs- und Laserquelle nach Anspruch 7, wobei die Dimmmechanismushalterung weiterhin einen Nocken (54) aufweist, der dazu ausgelegt ist, auf den zweiten Teil (50) den Druck anzulegen.

9. Beleuchtungsquelle nach Anspruch 1, wobei die Beleuchtungsquelle (36) eine Bogenlampe aufweist, die eine Halterung hat, die ein Ersetzen der Bogenlampe erlaubt und dennoch eine Platzierung einer Plasmakugel der Beleuchtungsquelle präzise an einer vorherbestimmten Stelle in einem optischen Zentrum beibehält.

10. Beleuchtungsquelle nach Anspruch 1, wobei die erste Linse (42) dazu ausgelegt ist, einen Abschnitt des Beleuchtungslichts in einen kollimierten Beleuchtungslichtpfad zu steuern, wobei das Steuern der ersten Linse (42) ein Dimmen des Beleuchtungslichts bereitstellt.

11. Beleuchtungs- und Laserquelle nach Anspruch 1, die weiterhin
einen Verbinder (98), der mit der optischen Faser (60) angebracht und dazu ausgelegt ist, anzuzeigen, ob das Laserlicht (14) oder das Beleuchtungslicht (11) oder beide durch die optische Faser (60) übertragen werden soll, und
eine Phototoxizitätsrisikokarte (76) aufweist, die dazu ausgelegt ist, eine Darstellung der Beleuchtungsrisiken während des Dimmens bereitzustellen,
wobei die erste Linse (42) dazu ausgelegt ist, einen Abschnitt des Beleuchtungslichts in einen kollimierten Beleuchtungslichtpfad zu steuern, wobei das Steuern der ersten Linse (42) ein Dimmen des Beleuchtungslichts bereitstellt.

12. Beleuchtungs- und Laserquelle nach Anspruch 1, die weiterhin eine Leistungsmesseinrichtung (150) mit einem Sensor (152) zur Aufnahme einer Lichtleistung, einer Anzeige (154) zum Anzeigen eines Werts der Lichtleistung und einem Steuerkreis (156) aufweist.

13. Beleuchtungs- und Laserquelle nach Anspruch 2, wobei der Lenkspiegel (24) einen Schatten bereitstellt, der den Laserstrahl in dem kollimierten Beleuchtungslichtpfad (11), in dem der kollimierte Laserstrahl (22) platziert ist, nicht auswäscht, und ein Ausgang der optischen Faser (60) dazu ausgelegt ist, ein oder mehrere interessierende Gewebe zu beleuchten, und der Schatten dort auf den Geweben erscheint, wo der Laserstrahl (22) für eine Behandlung platziert wird.

## Revendications

1. Source d'éclairage et de laser comprenant
une source d'éclairage (36) présentant une sortie de lumière d'éclairage à spectre large dans le domaine visible utile en chirurgie ;
une première lentille (42) appropriée pour collimater une partie de la lumière d'éclairage en un trajet de lumière d'éclairage collimaté (11) ; et
une source de lumière laser présentant une sortie de lumière laser (14), la lumière laser (14) étant collimatée pour former un faisceau laser collimaté (22) ; et
une seconde lentille (58) appropriée pour concentrer la lumière d'éclairage collimatée (11) et le faisceau laser collimaté (22) sur une fibre optique unique (60) ; dans laquelle
la source d'éclairage (36) présente un point focal d'éclairage d'une taille suffisamment faible pour coupler la lumière d'éclairage dans la fibre optique unique de sortie (60),
le faisceau laser collimaté (22) est positionné de manière à se trouver au sein du trajet (11) de lumière d'éclairage collimatée,
**caractérisée en ce que**
la fibre optique de sortie (60) présente un diamètre inférieur ou égal à 500 µm, et
un mécanisme de gradation est fourni pour grader la lumière d'éclairage au niveau de la sortie de la fibre optique (60).

2. Source d'éclairage et de laser selon la revendication 1, comprenant en outre
une lentille de collimation de laser (20) appropriée pour collimater ladite lumière laser (14) ; et
un miroir d'orientation (24) situé au sein d'un axe dudit trajet (11) de lumière d'éclairage collimatée et réfléchissant ledit faisceau laser collimaté (22), ce qui permet audit faisceau laser collimaté (22) d'être situé au sein du trajet (11) de lumière d'éclairage collimatée.

3. Source d'éclairage et de laser selon la revendication 2, dans laquelle ledit faisceau laser collimaté (22) au sein de ladite lumière d'éclairage collimatée (11) est essentiellement parallèle à ladite lumière d'éclairage (11).

4. Source d'éclairage et de laser selon la revendication 1, comprenant en outre
un ou plusieurs réflecteurs sphériques (40) présentant un centre géométrique (35) ; et
ladite source d'éclairage (36) étant située au niveau dudit centre géométrique (35) et au niveau d'un point focal de ladite première lentille (42).

5. Source d'éclairage et de laser selon la revendication 2, comprenant en outre
un ou plusieurs réflecteurs sphériques (40) présentant un centre géométrique (35) ; et
ladite source d'éclairage (36) étant située au niveau dudit centre géométrique (35) et au niveau d'un point focal de ladite première lentille (42).

6. Source d'éclairage et de laser selon la revendication 1, comprenant en outre
un mécanisme de gradation comprenant une monture (44) sur ladite première lentille (42) appropriée pour orienter ladite première lentille (42) dans une direction essentiellement perpendiculaire à un axe optique dudit trajet de lumière d'éclairage collimatée, ce qui permet à une ouverture numérique de lentille de ladite première lentille (42) de ne pas être essentiellement modifiée.

7. Source d'éclairage et de laser selon la revendication 6, ladite monture (44) de mécanisme de gradation comprenant en outre
une première pièce (48) fixée à un banc d'optique (12) et une seconde pièce (50) maintenant ladite première lentille (42), lesdites première et seconde pièces (48, 50) étant fixées à un ressort (52), ce qui permet à une pression exercée sur ladite seconde pièce (50) d'entrainer une déviation dudit ressort (52) et un déplacement de ladite première lentille (42) à se déplacer.

8. Source d'éclairage et de laser selon la revendication 7, ladite monture de mécanisme de gradation comprenant en outre :
une came (54) appropriée pour exercer ladite pression sur ladite seconde pièce (50).

9. Source d'éclairage et de laser selon la revendication 1, dans laquelle ladite source d'éclairage (36) comprend une lampe à arc présentant une monture qui permet un remplacement de ladite lampe à arc et conserve cependant de manière précise un emplacement d'une boule de plasma de ladite source d'éclairage au niveau d'un emplacement prédéterminé au sein d'un centre optique.

10. Source d'éclairage et de laser selon la revendication 1,
dans laquelle ladite première lentille (42) est appropriée pour orienter une partie de ladite lumière d'éclairage dans un trajet de lumière d'éclairage collimatée, l'orientation de ladite première lentille (42) fournissant une gradation de ladite lumière d'éclairage.

11. Source d'éclairage et de laser selon la revendication 1, comprenant en outre
un connecteur (98) monté avec ladite fibre optique (60) et approprié pour indiquer si ladite lumière laser (14) ou ladite lumière d'éclairage (11) ou les deux doivent être transmises à travers ladite fibre optique (60) ; et
une carte de risque de phototoxicité (76) appropriée pour fournir une représentation des risques d'éclairage pendant la gradation,
dans laquelle ladite première lentille (42) est appropriée pour orienter une partie de ladite lumière d'éclairage dans un trajet de lumière d'éclairage collimatée, l'orientation de ladite première lentille (42) fournissant une gradation de ladite lumière d'éclairage.

12. Source d'éclairage et de laser selon la revendication 1, comprenant en outre
un mesureur de puissance (150) présentant un capteur (152) destiné à recevoir une puissance lumineuse, un affichage (154) destiné à indiquer une valeur de ladite puissance lumineuse, et un circuit de commande (156).

13. Source d'éclairage et de laser selon la revendication 2, dans laquelle ledit miroir d'orientation (24) fournit une ombre qui n'affaiblit pas ledit faisceau laser au sein dudit trajet (11) de lumière d'éclairage collimatée là où est placé ledit faisceau laser collimaté (22) et une sortie de ladite fibre optique (60) est appropriée pour éclairer un ou plusieurs tissus d'intérêt et ladite ombre apparaît sur ledit ou lesdits tissu(s) là où ledit faisceau laser (22) est placé en vue d'un traitement.
